# EUROPEAN PATENT APPLICATION

(11) **EP 4 350 710 A1**
(43) Date of publication of application: **10.04.2024**
(21) Application number: 22879612.4
(22) Date of filing: 18.08.2022
(51) Int. Cl.: G16H 50/50, G16H 50/30, G16H 50/70, G16H 50/20, G01N 33/483, G01N 21/65

(54) **ARTIFICIAL INTELLIGENCE-BASED SIMULTANEOUS MULTI-CANCER DIAGNOSTIC SYSTEM USING EXOSOME SERS SIGNALS, AND METHOD THEREFOR**

(71) Applicant: Exopert Corporation, Seoul 02580 (KR)
(72) Inventor: SHIN, Hyunku, Seoul 02776 (KR); SHIM, On, Seoul 01460 (KR)
(74) Representative: Roos, Peter
(86) International application number: PCT/KR2022/012341
(87) International publication number: WO 2024/038927

(57) **Abstract**

The present invention relates to a multi-cancer simultaneous diagnosis system which is based on artificial intelligence and uses an exosome SERS signal and a method thereof.

According to the present invention, a multi-cancer simultaneous diagnosis system, which is based on artificial intelligence and uses an exosome SERS signals, includes a signal acquisition unit configured to drop exosomes acquired from a measurement target person on a chip to acquire a plurality of the exosome surface enhanced Raman spectroscopy (SERS) signals from the chip, a cancer diagnosis unit configured to acquire signal values of 0 or 1 for each of the plurality of exosome SERS signals by inputting the plurality of acquired exosome SERS signals to a trained cancer classification algorithm and configured to diagnose cancer or normality by using an average of the acquired signal values, and a cancer information providing unit configured to acquire the signal values of 0 or 1 for each of the plurality of exosome SERS signals by inputting the plurality of exosome SERS signals to a plurality of tissue of origin (TOO) determination algorithms when diagnosed as the cancer, configured to predict a cancer type by using the average of the acquired signal values, and configured to provides information on the predicted cancer type.

The present invention is a technology developed through "Commercialization of In Vitro Diagnostic Device for Simultaneous Screening of Multiple Cancers through Artificial Intelligence" that is Bio/Medical Technology Commercialization Support Project in 2021 (BT210040) of the Seoul Business Agency in Seoul.

## Description

### [Technical Field]

The present invention relates to a multi-cancer simultaneous diagnosis system which is based on artificial intelligence and uses exosome SERS signals and a method thereof, and more specifically, to a multi-cancer simultaneous diagnosis system and a method that detect a SERS signal map across the entire exosome rather than a specific marker and simultaneously diagnose multiple cancers through an artificial intelligence algorithm learned by using the detected SERS signal map.

### [Background Art]

Cancer is still a leading cause of death for modern people, but early diagnosis improves cancer outcomes by providing appropriate treatment. Early cancer detection by in vitro diagnosis is one of important goals in the field of biomedicine, and routine cancer management is possible.

However, several cancer biomarkers, such as carcinoembryonic antigen (CEA)5 and prostate-specific antigen (PSA)6, are currently being used for diagnosis and prognosis strategies of various cancers but are rarely present in early cancers, and there is a problem in that many cancers still suffer from the lack of effective pre-screening tools.

Recently, there have been intended to apply exosomes, which contain abundant information of parental cells, as a biomarker for liquid biopsy, and research has been conducted to diagnose or monitor diseases early by detecting information of disease exosomes existing in body fluids by applying various biomaterial detection technologies.

Conventionally, there has been an attempt to diagnose cancer by using SERS signals of exosomes in blood, but this is only a diagnosis of a single cancer, and multiple cancers may not be diagnosed at the same time, and there is a problem in that a false-positive diagnosis is made due to a classification error of an algorithm.

A background technology of the present invention is disclosed in Korean Patent No. 10-2225231 (Published on March 9, 2021).

### [Disclosure]

### [Technical Problem]

According to the present invention, a multi-cancer simultaneous diagnosis system that detects a SERS signal map across the entire exosome rather than a preset marker and simultaneously diagnoses multiple cancers through an artificial intelligence algorithm trained by using the detected SERS signal map and a method thereof are provided.

### [Technical Solution]

According to the present invention, a multi-cancer simultaneous diagnosis system, which is based on artificial intelligence and uses exosome SERS signals, includes a signal acquisition unit configured to drop exosomes acquired from a measurement target person on a chip to acquire a plurality of the exosome surface enhanced Raman spectroscopy (SERS) signals from the chip, a cancer diagnosis unit configured to acquire signal values of 0 or 1 for each of the plurality of exosome SERS signals by inputting the plurality of acquired exosome SERS signals to a trained cancer classification algorithm and configured to diagnose cancer or normality by using an average of the acquired signal values, and a cancer information providing unit configured to acquire the signal values of 0 or 1 for each of the plurality of exosome SERS signals by inputting the plurality of exosome SERS signals to a plurality of tissue of origin (TOO) determination algorithms when diagnosed as the cancer, configured to predict a cancer type by using the average of the acquired signal values, and configured to provides information on the predicted cancer type.

The multi-cancer simultaneous diagnosis system may further include a SERS signal collection unit configured to acquire a plurality of first exosome SERS signals from exosomes acquired from a normal person, configured to acquire a plurality of second exosome SERS signals from exosomes acquired from cancer patients having a plurality of cancer types, configured to label the plurality of first exosomes SERS signals as 0, and configured to label the plurality of second exosome SERS signals as 1, and a first learning unit configured to input the plurality of labeled first exosome SERS signals and the plurality of labeled second exosome SERS signals to a cancer classification algorithm to train the cancer classification algorithm to classify each of the plurality of input first and second exosome SERS signals as 0 or 1.

The multi-cancer simultaneous diagnosis system may further include a second learning unit configured to input the second exosome SERS signal acquired from a cancer patient having a preset cancer type among the plurality of cancer types and the second exosome SERS signals acquired from the other cancer patients excluding the cancer patient having the preset cancer type to the plurality of tissue of origin (TOO) determination algorithms to train each of the plurality of TOO determination algorithms to determine whether the second exosome SESR signals correspond to the preset cancer type.

The signal acquisition unit may acquire an exosome SERS signal map including n*m exosome SERS signals from the chip including n*m (where n and m are identical or different natural numbers) dot arrays.

The cancer diagnosis unit may input the n*m exosome SERS signals to the cancer classification algorithm and output signal values of 0 or 1 respectively corresponding to the n*m exosome SERS signals, and classify as normality when an average of the output signal values is close to 0 and classify as cancer when the average of the output signal values is close to 1.

The cancer information providing unit may input the n*m exosome SERS signals to the plurality of TOO determination algorithms, and the plurality of TOO determination algorithms may output signal values of 0 or 1 for each of the input n*m exosome SERS signals, and the cancer information providing unit may compare an average of the output signal values with a classification reference value for each cancer type to determine each cancer type.

A multi-cancer simultaneous diagnosis method using a multi-cancer simultaneous diagnosis system includes dropping exosomes acquired from a measurement target person on a chip to acquire a plurality of exosome surface enhanced Raman spectroscopy (SERS) signals from the chip, acquiring signal values of 0 or 1 for each of the plurality of exosome SERS signals by inputting the plurality of acquired exosome SERS signals to a trained cancer classification algorithm and diagnosing cancer or normality by using an average of the acquired signal values, and acquiring the signal values of 0 or 1 for each of the plurality of exosome SERS signals by inputting the plurality of exosome SERS signals to a plurality of tissue of origin (TOO) determination algorithms when diagnosed as the cancer, predicting a cancer type by using the average of the acquired signal values, and providing information on the predicted cancer type.

### [Advantageous Effects]

As described above, according to the present invention, an exosome SERS signal map is input to a cancer classification algorithm to first diagnose cancer, and thus, a false-positive diagnosis is reduced, and the multi-cancer simultaneous diagnosis system distinguishes cancer types by inputting the exosome SERS signal map diagnosed as cancer to a plurality of TOO determination algorithms and re-analyzing the exosome SERS signal map, and thereby, early cancer may be detected.

In addition, according to the present invention, limitations of conventional signal heterogeneity may be overcome by analyzing SERS signal of complex exosomes through artificial intelligence. Through this, non-invasive liquid biopsy of cancer due to exosomes may be possible. In addition, the multi-cancer simultaneous diagnosis system may diagnose cancer only with blood without risk of radiation exposure such as X-ray or CT, or invasive tissue biopsy and may be used not only for cancer diagnosis but also for treatment monitoring of patients.

### [Description of Drawings]

FIG. 1 is a configuration diagram illustrating a multi-cancer simultaneous diagnosis system according to an embodiment of the present invention.
FIG. 2 is a flowchart illustrating a multi-cancer simultaneous diagnosis method using a multi-cancer simultaneous diagnosis system, according to an embodiment of the present invention.
FIG. 3 is an example diagram illustrating SERS spectra appearing in each cancer type in step S210 illustrated in FIG. 2.
FIG. 4 is an example diagram illustrating a method of labeling a first exosome SERS signal and a second exosome SERS signal in step S220 illustrated in FIG. 2.
FIG. 5 is an example diagram illustrating a method of training a cancer classification algorithm in step S220 illustrated in FIG. 2.
FIG. 6 is an example diagram illustrating step S230 illustrated in FIG. 2.
FIG. 7 is an example diagram illustrating step S240 illustrated in FIG. 2.
FIG. 8 illustrates an example of accuracy of cancer diagnosis according to an embodiment of the present invention.
FIG. 9 illustrates example diagrams of a degree of false-positive diagnosis when cancer types are distinguished by using a tissue of origin (TOO) determination algorithm after a cancer patient is diagnosed, according to an embodiment of the present invention.
FIG. 10 illustrates examples of ROC curves for an artificial intelligence-based multi-cancer simultaneous diagnosis method using exosome SERS signals, according to an embodiment of the present invention.
FIG. 11 is an example diagram illustrating accuracy of multi-cancer diagnosis results using a TOO determination algorithm according to an embodiment of the present invention.

### [Best Mode]

Hereinafter, preferred embodiments according to the present invention will be described in detail with reference to the accompanying drawings. In this process, thicknesses of lines or sizes of components illustrated in the drawings may be enlarged for clarity and convenience of description.

In addition, terms to be described below are defined in consideration of functions in the present invention, which may change according to the intention or custom of a user or an operator. Therefore, definitions of the terms will have to be made based on the content throughout the present specification.

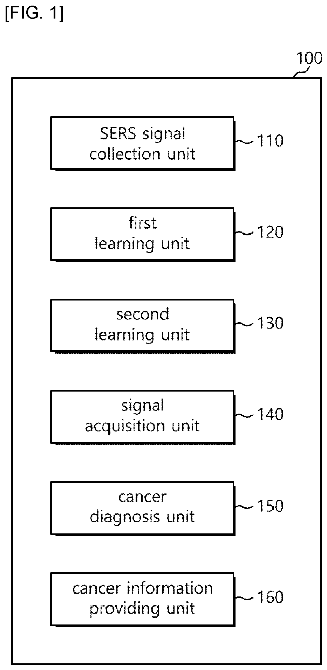

Hereinafter, a multi-cancer simultaneous diagnosis system according to an embodiment of the present invention will be described in more detail with reference to FIG. 1.

FIG. 1 is a configuration diagram illustrating a multi-cancer simultaneous diagnosis system according to an embodiment of the present invention.

As illustrated in FIG. 1, a multi-cancer simultaneous diagnosis system 100 according to an embodiment of the present invention includes a SERS signal collection unit 110, a first learning unit 120, a second learning unit 130, a signal acquisition unit 140, a cancer diagnosis unit 150, and a cancer information providing unit 160.

First, the SERS signal collection unit 110 collects exosome SERS signals acquired by performing Raman spectroscopy on an exosome collected from blood plasma of normal person and an exosome collected from blood plasma of a cancer patient.

Hereinafter, for the sake of convenience of description, an exosome SERS signal acquired from a normal person is referred to as a first exosome SERS signal, and an exosome SERS signal acquired from a cancer patient is referred to as a second exosome SERS signal.

In addition, the SERS signal collection unit 110 labels a plurality of first exosome SERS signals as 0 and labels a plurality of second exosome SERS signals as 1.

The first learning unit 120 builds a cancer classification algorithm based on deep learning and inputs the labeled first exosome SERS signal and the labeled second exosome SERS signal to the built cancer classification algorithm to perform training. Then, the cancer classification algorithm outputs a signal value of 0 or 1 in response to the input first and second exosome SERS signals.

The second learning unit 130 builds a tissue of origin (TOO) determination algorithm based on deep learning. In this case, a plurality of TOO determination algorithms are formed to correspond to a cancer type. Here, the cancer type may include at least one of lung cancer, breast cancer, colorectal cancer, liver cancer, pancreas cancer, and stomach cancer but is not limited thereto and may include various other cancer types.

Next, the second learning unit 130 inputs the second exosome SERS signal acquired from each cancer patient into the plurality of TOO determination algorithms to perform training. Here, the TOO determination algorithm for a preset cancer type receives the second exosome SERS signal acquired from a cancer patient corresponding to the preset cancer type and the second exosome SERS signals acquired from the other cancer patients excluding the cancer patient of the preset cancer type and is learned to determine whether the exosome SESR signals correspond to a preset cancer type. Then, the TOO determination algorithm outputs a signal value of 0 or 1 for an input exosome SERS signal.

The signal acquisition unit 140 drops exosomes collected from blood plasma of a measurement target person onto a chip, and then performs Raman spectroscopy on the chip to acquire a plurality of exosomes SERS signals.

The cancer diagnosis unit 150 acquires a signal value of 0 or 1 for each exosome SERS signal by inputting a plurality of acquired exosome SERS signals to the cancer classification algorithm for which training is completed. Then, the cancer diagnosis unit 150 diagnoses the measurement target person as a normal person or a cancer patient by using an average of the acquired signal values.

When the measurement target person is diagnosed as a cancer patient, the cancer information providing unit 160 inputs a plurality of exosome SERS signals respectively to a plurality of TOO determination algorithms for which training is completed.

Then, each of the plurality of TOO determination algorithms outputs a signal value of 0 or 1 for each of the plurality of input exosome SERS signals, and the cancer information providing unit 160 compares an average of the output signal values with a classification reference value for a corresponding cancer type to determine whether the exosome SERS signals correspond to each cancer type.

For example, a lung cancer TOO determination algorithm outputs signal values of 0 or 1 for a plurality of input exosome SERS signals and compares an average of the output signal values with a lung cancer classification reference value to determine whether there is cancer. A breast cancer TOO determination algorithm outputs signal values of 0 or 1 for a plurality of input exosome SERS signals and compares an average of the output signal values with a breast cancer classification reference value to determine whether there is breast cancer. A colorectal cancer TOO determination algorithm outputs signal values of 0 or 1 for a plurality of input exosome SERS signals, and compares an average of the output signal values with a colorectal cancer classification reference value to determine whether there is colorectal cancer. A liver cancer TOO determination algorithm outputs signal values of 0 or 1 for a plurality of input exosome SERS signals and compares an average of the output signal values with a liver cancer classification reference value to determine whether there is liver cancer. A pancreas cancer TOO determination algorithm outputs signal values of 0 or 1 for a plurality of input exosome SERS signals and compares an average of the output signal values with a pancreas cancer classification reference value to determine whether there is pancreas cancer. Finally, a stomach cancer TOO determination algorithm outputs signal values of 0 or 1 for a plurality of input exosome SERS signals and compares an average of the output signal values with a stomach cancer classification reference value to determine whether there is stomach cancer.

In addition, the cancer information providing unit 160 provides information on a cancer type determined as benign.

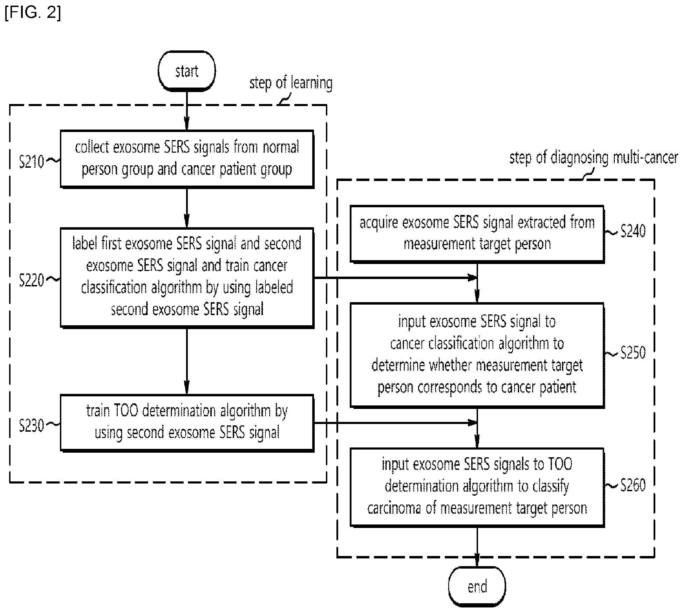

A multi-cancer simultaneous diagnosis method using a multi-cancer simultaneous diagnosis system, according to an embodiment of the present invention, will be described in more detail with reference to FIGS. 2 to 7.

FIG. 2 is a flowchart illustrating a multi-cancer simultaneous diagnosis method using a multi-cancer simultaneous diagnosis system, according to an embodiment of the present invention.

As illustrated in FIG. 2, the multi-cancer simultaneous diagnosis method using the multi-cancer simultaneous diagnosis system, according to an embodiment of the present invention, includes a step of training a cancer classification algorithm and a TOO determination algorithm and a step of diagnosing multiple cancers by using the trained cancer classification algorithm and the trained TOO determination algorithm.

First, in the step of training the cancer classification algorithm and the TOO determination algorithm, The multi-cancer simultaneous diagnosis system 100 collects exosome SERS signals from a normal person group and a cancer patient group (S210).

More specifically, a blood plasma sample is acquired from a normal person, and exosomes are isolated from the acquired blood plasma sample by using size exclusion chromatography (SEC). In addition, exosomes are isolated from a blood plasma samples of a cancer patient in the same manner.

Then, an exosome solution acquired by being isolated from the blood plasma sample of a normal person and an exosome solution acquired by being isolated from a blood plasma sample of a cancer patient are dropped on each Au nanoparticle assembly array chip and then dried.

Here, the Au nanoparticle assembly array chip is formed by precipitating Au nanoparticles (AuNPs) in a colloidal solution and then coating the NPs on an APTES-functionalized glass surface. In order to increase detection throughput and uniformity of a signal acquisition process on the APTES-functionalized glass surface, the Au nanoparticle assembly array chip includes n*m (where n and m are the identical or different natural numbers) dot arrays, and an exosome SERS signal is measured in each dot.

Then, the SERS signal collection unit 110 collects an exosome signal map including a plurality of exosome SERS signals corresponding to the dot arrays by performing the Raman spectroscopy on the Au nanoparticle assembly array chip in which the exosome solution is dried.

That is, the SERS signal collection unit 110 collects n*m first exosome SERS signals from an exosome solution of a normal person and collects n*m second exosome SERS signals from an exosome solution of a cancer patient.

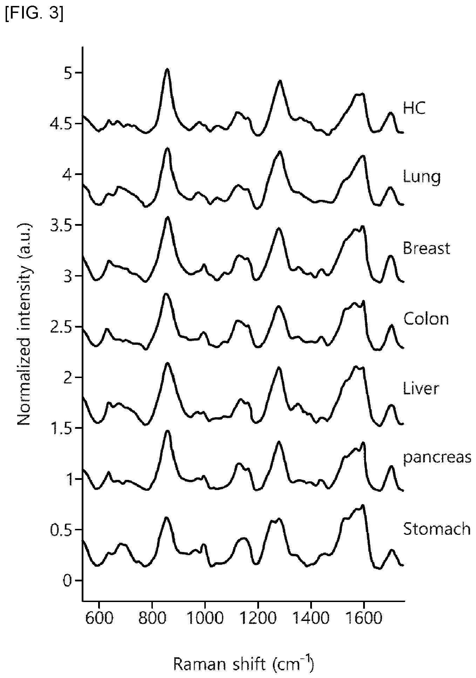

FIG. 3 is an example diagram illustrating SERS spectra appearing in each cancer type in step S210 illustrated in FIG. 2.

The multi-cancer simultaneous diagnosis system 100 according to the embodiment of the present invention configures a cancer patient group by using patients diagnosed with at least one cancer among lung cancer, breast cancer, colorectal cancer, liver cancer, pancreas cancer, and stomach cancer.

As illustrated in FIG. 3, the lung cancer, the breast cancer, the colorectal cancer, the liver cancer, the pancreas cancer, and the stomach cancer have different SERS spectra.

When step S210 is completed, the first learning unit 120 labels the first exosome SERS signal acquired from a normal person group and the second exosome SERS signal acquired from a cancer patient group and trains a cancer classification algorithm by using the labeled first and second exosome SERS signals (S220).

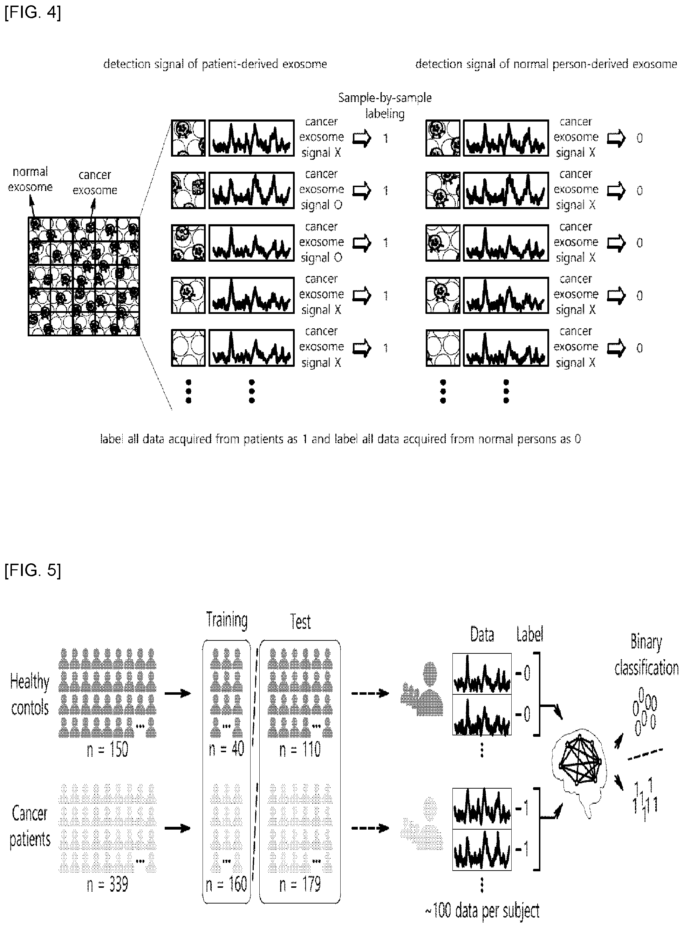

FIG. 4 is an example diagram illustrating a method of labeling the first exosome SERS signal and the second exosome SERS signal in step S220 illustrated in FIG. 2.

As illustrated in FIG. 4, an exosome solution of a cancer patient includes both normal exosomes and cancer exosomes, and accordingly, n*m dots arranged in the Au nanoparticle assembly array chip may include either the normal exosomes or the cancer exosomes and may also include both the normal exosomes and the cancer exosomes. That is, a plurality of exosome SERS signals respectively corresponding to the dot arrays may be output differently.

However, the first learning unit 120 according to an embodiment of the present invention does not classify the exosome SERS signals according to whether there are cancer exosomes or normal exosomes and labels a plurality of first exosome SERS signals acquired from a normal person as 0 and labels a plurality of second exosome SERS signals acquired from a cancer patient as 1.

FIG. 5 is an example diagram illustrating a method of training a cancer classification algorithm in step S220 illustrated in FIG. 2.

As illustrated in FIG. 5, the first learning unit 120 randomly extracts learning data and test data from the labeled first and second exosome SERS signals.

Then, the first learning unit 120 trains a cancer classification algorithm by using the first exosome SERS signal and the second exosome SERS signal corresponding to the extracted learning data as input data and using the labeled values as output data.

That is, the cancer classification algorithm outputs signal values of 0 or 1 in response to a plurality of input exosome SERS signals and first diagnoses whether there is cancer by using an average of the output signal values.

Next, the second learning unit 130 trains a TOO determination algorithm by using the second exosome SERS signal of the cancer patient group acquired in step S210 (S230).

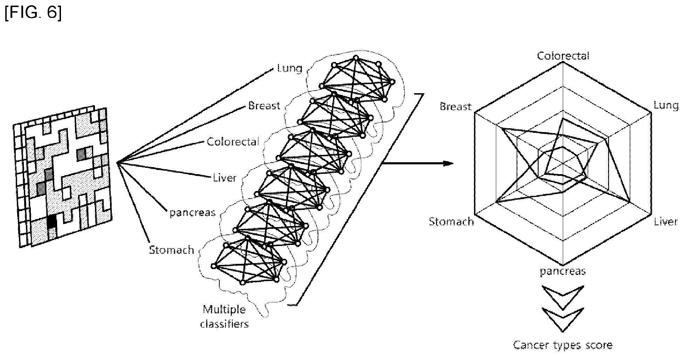

FIG. 6 is an example diagram illustrating step S230 illustrated in FIG. 2.

As illustrated in FIG. 6, the second learning unit 130 constructs a plurality of TOO determination algorithms respectively corresponding to lung cancer, breast cancer, colorectal cancer, liver cancer, pancreas cancer, and stomach cancer.

Then, the second learning unit 130 inputs the second exosome SERS signals acquired from a lung cancer patient group and the second exosome SERS signals acquired from the other cancer patient groups excluding the lung cancer patient group to train a lung cancer TOO determination algorithm to output a signal value of 0 or 1 corresponding to the exosome SERS signal which is input.

In addition, the second learning unit 130 inputs the second exosome SERS signals acquired from a breast cancer patient group and the second exosome SERS signals acquired from the other cancer patient groups excluding the breast cancer patient group to a breast cancer TOO determination algorithm to train the breast cancer TOO determination algorithm to output a signal value of 0 or 1 correspond to the exosome SERS signal which is input.

The second learning unit 130 also trains a colorectal cancer TOO determination algorithm, a liver cancer TOO determination algorithm, a pancreas cancer TOO determination algorithm, and a stomach TOO determination algorithm in the same way.

When the training of the algorithms is completed by using step S210 to step S230, the multi-cancer simultaneous diagnosis system 100 diagnoses multiple cancers for a measurement target person.

First, the signal acquisition unit 140 acquires an exosome SERS signal extracted from blood plasma of the measurement target person (S240).

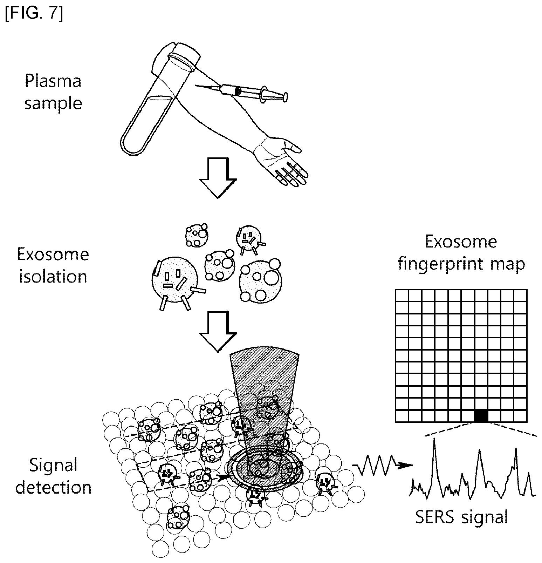

FIG. 7 is an example diagram illustrating step S240 illustrated in FIG. 2.

As illustrated in FIG. 7, a user collects blood plasma from a measurement target person and isolates exosomes from the collected blood plasma by applying chromatography to the collected blood plasma.

Then, the user drops an exosome-dissolved solution on an Au nanoparticle assembly array chip and then dries the exosome-dissolved solution.

Then, Raman spectroscopy is performed on the Au nanoparticle assembly array chip to acquire n*m (for example, 100) exosome SERS signals corresponding to dot arrays.

When step S240 is completed, the cancer diagnosis unit 150 inputs the n*m exosome SERS signals to a cancer classification algorithm to determine whether the measurement target person corresponds to a cancer patient (S250).

More specifically, the cancer diagnosis unit 150 inputs the n*m exosome SERS signals to a cancer classification algorithm. Then, the cancer classification algorithm outputs signal values of 0 or 1 respectively corresponding to the n*m exosome SERS signals.

When an average of the output signal values is close to 0, the cancer diagnosis unit 150 diagnoses the measurement target person as a normal person, and when the average of the output signal values is close to 1, the cancer diagnosis unit 150 diagnoses the measurement target person as a cancer patient.

When the measurement target person is diagnosed as a cancer patient in step S250, the cancer information providing unit 160 inputs the n*m exosome SERS signals to a plurality of trained TOO determination algorithms to classify a cancer type of the measurement target person (S260).

More specifically, the cancer information providing unit 160 inputs the n*m exosome SERS signals respectively to a lung cancer TOO determination algorithm, a breast cancer TOO determination algorithm, a colorectal cancer TOO determination algorithm, a liver cancer TOO determination algorithm, a pancreas cancer TOO determination algorithm, and a stomach cancer TOO determination algorithm.

Then, the lung cancer TOO determination algorithm outputs signal values of 0 or 1 for each of the input n*m exosome SERS signals and compares an average of the output signal values with a lung cancer classification reference value to determine whether a measurement target person corresponds to a lung cancer patient.

The breast cancer TOO determination algorithm outputs signal values of 0 or 1 for each of the input n*m exosome SERS signals and compares an average of the output signal values with a breast cancer classification reference value to determine whether a measurement target person corresponds to a breast cancer patient.

The colorectal cancer TOO determination algorithm outputs signal values of 0 or 1 for each of the input n*m exosome SERS signals and compares an average of the output signal values with a colorectal cancer classification reference value to determine whether a measurement target person corresponds to a colorectal cancer patient.

The liver cancer TOO determination algorithm outputs signal values of 0 or 1 for each of the input n*m exosome SERS signals and compares an average of the output signal values with a liver cancer classification reference value to determine whether a measurement target person corresponds to a liver cancer patient.

The pancreas cancer TOO determination algorithm outputs signal values of 0 or 1 for each of the input n*m exosome SERS signals and compares an average of the output signal values with a pancreas cancer classification reference value to determine whether a measurement target person corresponds to a pancreas cancer patient.

Finally, the stomach cancer TOO determination algorithm outputs signal values of 0 or 1 for each of the input n*m exosome SERS signals, and compares an average of the output signal values with a stomach cancer classification reference value to determine whether a measurement target person corresponds to a stomach cancer patient.

Next, the cancer information providing unit 160 provides a cancer type prediction result obtained by using the results output from the six TOO determination algorithms.

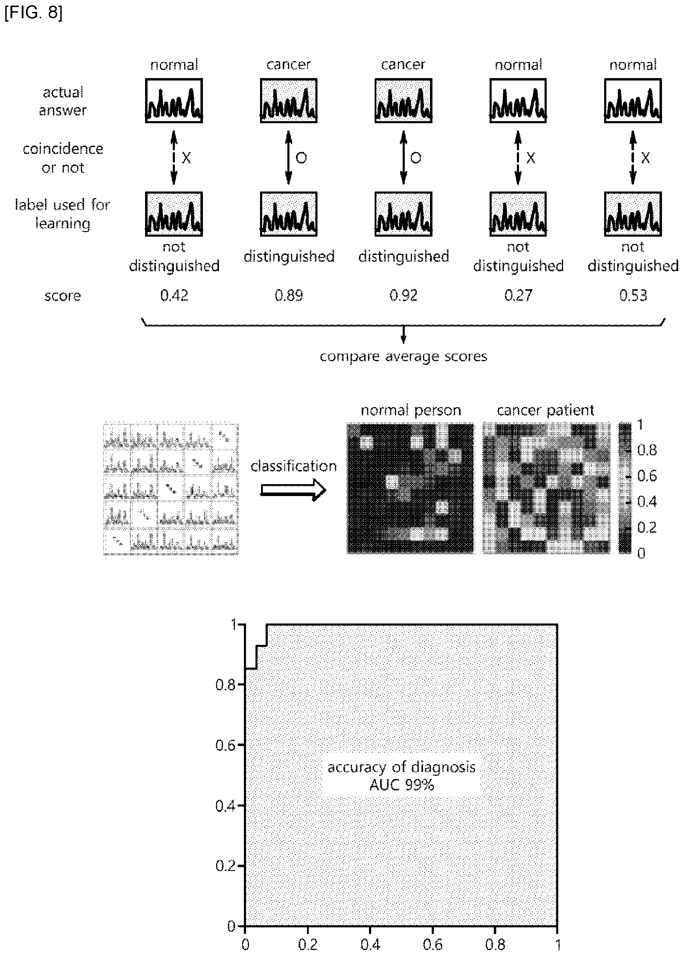

Hereinafter, results of simultaneously diagnosing multiple cancers by using the cancer classification algorithm and the TOO determination algorithms according to an embodiment of the present invention will be described in detail with reference to FIGS. 8 to 11.

FIG. 8 illustrates an example of accuracy of cancer diagnosis according to an embodiment of the present invention.

As illustrated in FIG. 8, when all exosome SERS signals acquired from a cancer patient are labeled as 1, the exosome SERS signals acquired in a region where there are no cancer exosome may differ from an actual answer. That is, accuracy of prediction for individual exosome SERS signals may be slightly reduced.

However, when a plurality of exosome SERS signals acquired from cancer patients are diagnosed by using an average of signal values output by a cancer classification algorithm, the diagnosis has accuracy of approximately 99%.

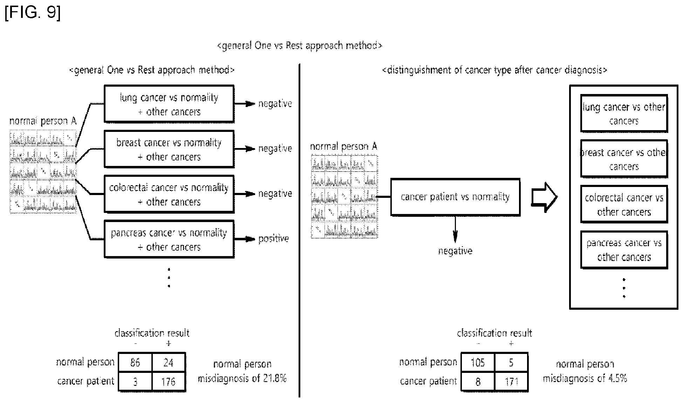

FIG. 9 illustrates example diagrams of a degree of false-positive diagnosis when cancer types are distinguished by using a TOO determination algorithm after a cancer patient is diagnosed, according to an embodiment of the present invention.

As illustrated in FIG. 9, when comparing the known One vs Rest approach method with an approach method of distinguishing a cancer type after cancer diagnosis according to an embodiment of the present invention, the One vs Rest approach method has a misdiagnosis rate of approximately 21.8%, but the approach method of distinguishing a cancer type after cancer diagnosis has a misdiagnosis rate of approximately 4.5%.

Therefore, the multi-cancer simultaneous diagnosis system according to the embodiment of the present invention has an effect of greatly reducing the probability of a false-positive diagnosis.

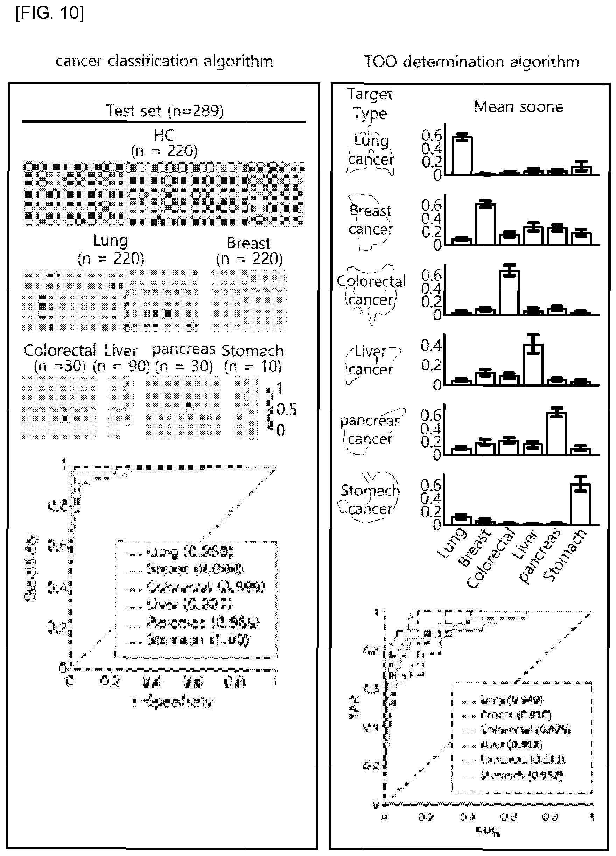

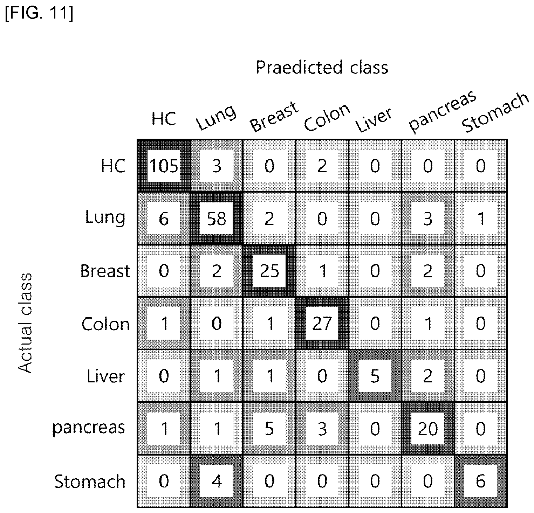

FIG. 10 illustrates examples of ROC curves for an artificial intelligence-based multi-cancer simultaneous diagnosis method using exosome SERS signals, according to an embodiment of the present invention, and FIG. 11 is an example diagram illustrating accuracy of multi-cancer diagnosis results using a TOO determination algorithm according to an embodiment of the present invention.

Specifically, FIG. 10 illustrates receiver operation characteristic (ROC) curves for verifying effects of a cancer classification algorithm and a TOO determination algorithm according to an embodiment of the present invention. The closer the area under curve (ROC curve) (AUC) is to 1, the greater the usefulness is, and the cancer classification algorithm according to the embodiment of the present invention shows a value of approximately 0.983, and a plurality of TOO determination algorithms also show excellent performance.

In addition, as illustrated in FIG. 11, it can be seen that a concordance rate of an actual diagnosis result and a prediction result according to the embodiment of the present invention is relatively high, and particularly, it can be seen that even early cancer patients may be predicted relatively accurately.

As such, the multi-cancer simultaneous diagnosis system according to the present invention inputs an exosome SERS signal map including a plurality of exosome SERS signals to a cancer classification algorithm to first diagnose cancer, and thus, a false-positive diagnosis is reduced, and the multi-cancer simultaneous diagnosis system distinguishes cancer types by inputting the exosome SERS signal map diagnosed as cancer to a plurality of TOO determination algorithms and re-analyzing the exosome SERS signal map, and thereby, early cancer may be detected.

In addition, the multi-cancer simultaneous diagnosis system according to the present invention may overcome limitations of conventional signal heterogeneity by analyzing SERS signal of complex exosomes through artificial intelligence. Through this, non-invasive liquid biopsy of cancer due to exosomes may be possible. In addition, the multi-cancer simultaneous diagnosis system may diagnose cancer only with blood without risk of radiation exposure such as X-ray or CT, or invasive tissue biopsy and may be used not only for cancer diagnosis but also for treatment monitoring of patients.

Although the present invention is described with reference to the embodiments illustrated in the drawings, this is only an example, and those skilled in the art will understand that various modifications and equivalent other embodiments are possible therefrom. will be. Therefore, the true technical protection scope of the present invention should be determined by the technical idea of the claims below.

## Claims

1. A multi-cancer simultaneous diagnosis system which is based on artificial intelligence and uses exosome SERS signals, the multi-cancer simultaneous diagnosis system comprising:
a signal acquisition unit configured to drop exosomes acquired from a measurement target person on a chip to acquire a plurality of the exosome surface enhanced Raman spectroscopy (SERS) signals from the chip;
a cancer diagnosis unit configured to acquire signal values of 0 or 1 for each of the plurality of exosome SERS signals by inputting the plurality of acquired exosome SERS signals to a trained cancer classification algorithm and configured to diagnose cancer or normality by using an average of the acquired signal values; and
a cancer information providing unit configured to acquire the signal values of 0 or 1 for each of the plurality of exosome SERS signals by inputting the plurality of exosome SERS signals to a plurality of tissue of origin (TOO) determination algorithms when diagnosed as the cancer, configured to predict a cancer type by using the average of the acquired signal values, and configured to provides information on the predicted cancer type.

2. The multi-cancer simultaneous diagnosis system of claim 1, further comprising:
a SERS signal collection unit configured to acquire a plurality of first exosome SERS signals from exosomes acquired from a normal person, configured to acquire a plurality of second exosome SERS signals from exosomes acquired from cancer patients having a plurality of cancer types, configured to label the plurality of first exosomes SERS signals as 0, and configured to label the plurality of second exosome SERS signals as 1; and
a first learning unit configured to input the plurality of labeled first exosome SERS signals and the plurality of labeled second exosome SERS signals to a cancer classification algorithm to train the cancer classification algorithm to classify each of the plurality of input first and second exosome SERS signals as 0 or 1.

3. The multi-cancer simultaneous diagnosis system of claim 2, further comprising:
a second learning unit configured to input the second exosome SERS signal acquired from a cancer patient having a preset cancer type among the plurality of cancer types and the second exosome SERS signals acquired from the other cancer patients excluding the cancer patient having the preset cancer type to the plurality of tissue of origin (TOO) determination algorithms to train each of the plurality of TOO determination algorithms to determine whether the second exosome SESR signals correspond to the preset cancer type.

4. The multi-cancer simultaneous diagnosis system of claim 1, wherein
the signal acquisition unit acquires an exosome SERS signal map including n*m exosome SERS signals from the chip including n*m (where n and m are identical or different natural numbers) dot arrays.

5. The multi-cancer simultaneous diagnosis system of claim 4, wherein
the cancer diagnosis unit inputs the n*m exosome SERS signals to the cancer classification algorithm and outputs signal values of 0 or 1 respectively corresponding to the n*m exosome SERS signals, and classifies as normality when an average of the output signal values is close to 0 and classifies as cancer when the average of the output signal values is close to 1.

6. The multi-cancer simultaneous diagnosis system of claim 5, wherein
the cancer information providing unit inputs the n*m exosome SERS signals to the plurality of TOO determination algorithms, and the plurality of TOO determination algorithms output signal values of 0 or 1 for each of the input n*m exosome SERS signals, and the cancer information providing unit compares an average of the output signal values with a classification reference value for each cancer type to determine each cancer type.

7. A multi-cancer simultaneous diagnosis method using a multi-cancer simultaneous diagnosis system, the multi-cancer simultaneous diagnosis method comprising:
dropping exosomes acquired from a measurement target person on a chip to acquire a plurality of exosome surface enhanced Raman spectroscopy (SERS) signals from the chip;
acquiring signal values of 0 or 1 for each of the plurality of exosome SERS signals by inputting the plurality of acquired exosome SERS signals to a trained cancer classification algorithm and diagnosing cancer or normality by using an average of the acquired signal values; and
acquiring the signal values of 0 or 1 for each of the plurality of exosome SERS signals by inputting the plurality of exosome SERS signals to a plurality of tissue of origin (TOO) determination algorithms when diagnosed as the cancer, predicting a cancer type by using the average of the acquired signal values, and providing information on the predicted cancer type.
